# EUROPÄISCHE PATENTSCHRIFT

(11) **EP 0 878 174 B1**
(45) Veröffentlichungstag und Bekanntmachung des Hinweises auf die Patenterteilung: **27.02.2002**
(21) Anmeldenummer: 98108278.7
(22) Anmeldetag: 06.05.1998
(51) Int. Cl.: A61F 2/06

(54) **Koronarer Stent**
Coronary stent
Extenseur coronaire

(30) Priorität: 15.05.1997 DE 29708689 U
(43) Veröffentlichungstag der Anmeldung: 18.11.1998
(73) Patentinhaber: Jomed Implantate GmbH, 72414 Rangendingen (DE)
(72) Erfinder: Van Oepen, Randolf, Dr. Ing., 72145 Hirrlingen (DE)
(74) Vertreter: Schmitz, Hans-Werner, Dipl.-Ing.

(56) Entgegenhaltungen:
- DE-U- 29 702 671
- US-A- 5 449 373
- US-A- 5 669 932

## Beschreibung

Die Erfindung bezieht sich auf einen koronaren Stent, mit einem rohrförmigen, flexiblen Körper, dessen Wand eine Stegstruktur aufweist, wobei die Stegstruktur mehrere zueinander benachbarte, durch Steg begrenzte Zellen aufweist, welche jeweils über gemeinsame Stege mit benachbarten Zellen verbunden sind, nach dem Oberbegriff des Anspruchs 1. Ein derartiger Stent ist aus der US 5,449,373 bekannt. Ähnliche stents sind auch aus der DE-U-297 02671 und der DE-U-297 01758 bekannt, die insbesondere V-förmig gefaltete stege enthalten.

Aus dem Stand der Technik sind unterschiedlichste Ausgestaltungsformen von koronaren Stents vorbekannt. Diese bilden eine Gefäßprothese, die aus körperverträglichem Material besteht. Der Stent bzw. die Stentprothese wird dazu verwendet, Blutgefäße oder aber auch andere Körperöffnungen aufzuweiten und in dem aufgeweiteten Zustand zu halten. Zu diesem Zwecke wird der Stent in einem nicht-expandierten Zustand im Körper des Patienten positioniert und nachfolgend durch geeignete Mittel, beispielsweise einen Ballonkatheter expandiert. Bei dem Expandieren werden die einzelnen Stegbereiche des Stents verformt, so daß dieser dauerhaft in der expandierten Form verbleibt.

Bei der Konstruktion von Stents ergibt sich grundsätzlich das Problem, daß diese im nicht-expandierten Zustand einen ausreichend kleinen Durchmesser aufweisen müssen, um in den Körper des Patienten eingebracht und dort positioniert werden zu können. Die Stents müssen dabei eine gewisse Flexibilität längs ihrer Längsachse haben, um den Formen von beispielsweise Blutgefäßen folgen zu können. Bei der Expansion muß der Stent auf einen wesentlich größeren Außendurchmesser aufgeweitet werden. Dies muß durch Verformung der einzelnen Stegbereiche so erfolgen, daß Rißbildungen oder ähnliches vermieden werden.

Der Erfindung liegt die Aufgabe zugrunde, einen Stent der eingangs genannten Art zu schaffen, welcher bei einfachem Aufbau, einfacher Herstellbarkeit und sicherer Anwendbarkeit expandierbar ist.

Erfindungsgemäß wird die Aufgabe durch die Merkmale des Hauptanspruchs gelöst, die Unteransprüche zeigen weitere vorteilhafte Ausgestaltungen der Erfindung.

Erfindungsgemäß ist somit vorgesehen, daß in Achsrichtung des Stents alternierend erste und zweite, voneinander verschiedene Zellen angeordnet sind, daß in Umfangsrichtung benachbart jeweils Gleichzellen vorgesehen sind, daß die ersten Zellen im nicht-expandierten Zustand des Stents nur an zwei gegenüberliegenden Seiten gefaltete Stege aufweisen und daß die zweiten Zellen im nicht-expandierten Zustand an allen Seiten mit ahwechseend, gefalteten und/oder aneinandergelegten Stegbereichen versehen sind, wobei die gefalteten stege der ersten zelten V-förmig angeordnet sind, die stege der zweiten Zellen zweier gegenüberliegender Seiten V-formig ausgebildet sind und die beiden anderen gegenüberliegenden stege bogen förmig aneinander angelegt sind.

Der erfindungsgemäße Stent zeichnet sich durch eine Reihe erheblicher Vorteile aus.

Die alternierende Anordnung unterschiedlicher Zellen ergibt im nicht-expandierten Zustand zum einen eine ausreichende Festigkeit, zum anderen aber auch eine hinreichende Flexibilität. Da die Stege oder Stegbereiche der unterschiedlichen Zellen ein unterschiedliches Expansionsverhalten aufweisen, kann der Stent in einfacher und zuverlässiger Weise expandiert werden. Die unterschiedlichen Zellen erlauben hierbei eine entsprechende Verformung der Stege, so daß Rißbildungen oder ähnliches ausgeschlossen werden können.

Die gefalteten Stege der ersten Zellen sind-förmig ausgestaltet, so daß diese bevorzugterweise einen sich in Umfangsrichtung erstreckenden bandartigen Bereich bilden. Diese bandartigen Bereiche erhöhen die Festigkeit des Stents und sichern auch im expandierten Zustand seine Formbeständigkeit.

Durch die unterschiedliche Ausgestaltung der Stege bzw. Stegbereiche der zweiten Zellen wird ein unterschiedliches Expansionsverhalten derselben ermöglicht, während die V-förmig ausgebildeten Stegbereiche ähnlich wie bei den ersten Zellen deformierbar sind, gestatten die bogenförmig aneinandergelegten Stegbereiche ein hohes Maß von Verformung und Expansion. Durch die bevorzugterweise s-förmige Anordnung können sehr lange Stegbereiche platzsparend im nicht-expandierten Zustand vorgesehen sein.

Die ersten Zellen weisen auch nicht-gefaltete Stege auf, die bevorzugterweise bogenförmig und zueinander parallel angeordnet sind. Auch dies führt zu einer ausreichenden Verformbarkeit der Zellen sowie zur Erhöhung der Stabilität, insbesondere im expandierten Zustand.

Im folgenden wird die Erfindung anhand eines Ausführungsbeispiels in Verbindung mit der Zeichnung beschrieben.

Dabei zeigt:
Fig. 1 eine schematisch stark vereinfachte Darstellung des Grundaufbaus des erfindungsgemäßen Stents,
Fig. 2 eine Darstellung der Stegstruktur der Wand des Stents im nicht-expandierten Zustand,
Fig. 3 eine vergrößerte Darstellung einer ersten Zelle, und
Fig. 4 eine vergrößerte Darstellung einer zweiten Zelle.

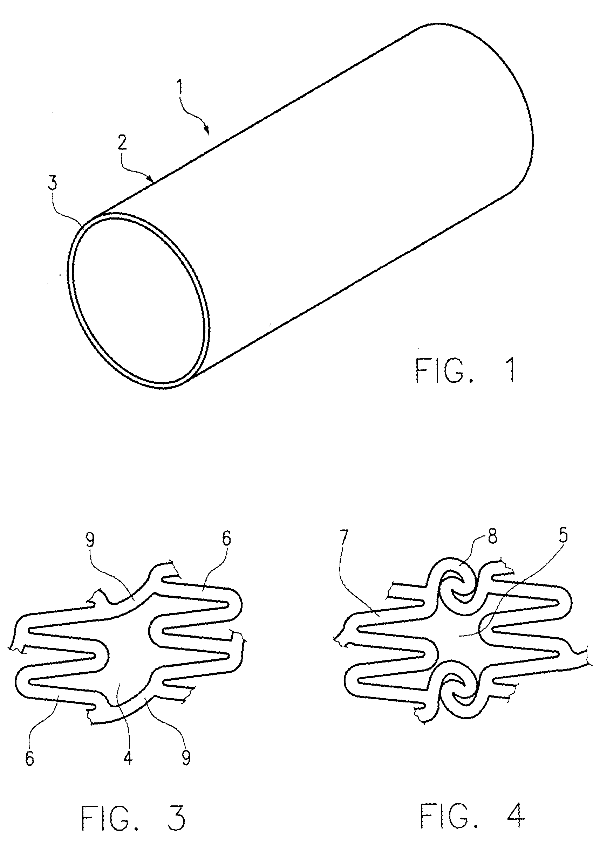

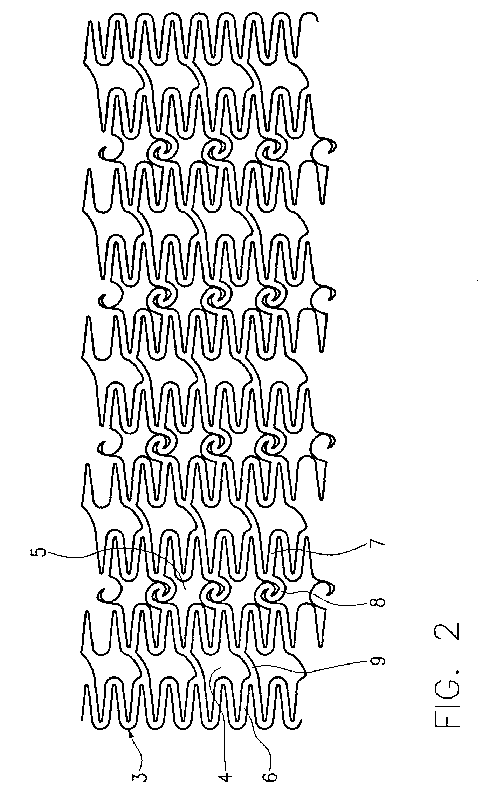

Die Fig. 1 zeigt den grundsätzlichen Aufbau eines erfindungsgemäßen Stents 1, der einen flexiblen, rohrförmigen Körper 2 mit einer Wand 3 aufweist, von der in Fig. 1 die Stirnansicht dargestellt ist.

In Fig. 2 ist der Aufbau der erfindungsgemäßen Stegstruktur gezeigt.

Aus den Figuren ergibt sich, daß in Umfangsrichtung abwechselnd jeweils Reihen von ersten Zellen 4 und von angrenzenden zweiten Zellen 5 vorgesehen sind. Die ersten Zellen 4 weisen jeweils an zwei gegenüberliegenden Seiten v-förmig angeordnete Stege 6 auf, während die anderen Seiten der ersten Zellen 4 durch zueinander parallele, bogenförmige Stege 9 gebildet werden.

Die Form und Ausgestaltung der zweiten Zellen 5 unterscheidet sich von den ersten Zellen 4 dadurch, daß sämtliche, die Zelle 5 begrenzenden Stege gefaltet oder aneinandergelegt sind.

Wie sich aus Fig. 4 ergibt, sind an zwei gegenüberliegenden Seiten der Zelle 5 v-förmige Stege 7 ausgebildet, während an den beiden anderen, gegenüberliegenden Bereichen Stege 8 vorgesehen sind, die bogenförmig ineinandergreifend ausgestaltet sind. Die Stege 8 weisen somit eine s-förmige Struktur auf.

Der erfindungsgemäß aufgebaute koronare Stent 1 weist sowohl in Längs- als auch in Querrichtung im nicht-expandierten Zustand eine gute Flexibilität auf. Die Anordnung und Ausgestaltung der Stege ergibt ein gutes Expansionsverhalten sowie ein hohes Maß an Formstabilität im expandierten Zustand.

Der erfindungsgemäße Stent ist aus dem körperverträglichen Material, insbesondere nicht-rostendem Stahl gefertigt. Die Erfindung ist nicht auf das gezeigte Ausführungsbeispiel beschränkt, vielmehr ergeben sich im Rahmen der Erfindung vielfältige Abwandlungs- und Modifikationsmöglichkeiten.

## Patentansprüche

1. Koronarer Stent (1) mit einem rohrförmigen, flexiblen Körper (2), dessen Wand eine Stegstruktur aufweist, wobei die Stegstruktur mehrere zueinander benachbarte, durch Stege begrenzte Zellen (4, 5) aufweist, welche jeweils über gemeinsame Stege mit benachbarten Zellen verbunden sind, wobei in Achsrichtung des Stents (1) alternierend erste (4) und zweite (5), voneinander verschiedene Zellen angeordnet sind, wobei in Umfangsrichtung benachbart jeweils gleiche Zellen vorgesehen sind, wobei die ersten Zellen (4) im nicht-expandierten Zustand des Stents (1) nur an zwei gegenüberliegenden Seiten gefaltete Stege (6) aufweisen und wobei die zweiten Zellen (5) im nicht-expandierten Zustand an allen Seiten mit abwechselnd unterschiedlich gefalteten und/oder aneinandergelegten Stegbereichen (7, 8) versehen sind, **dadurch gekennzeichnet, daß** die gefalteten Stege (6) der ersten Zellen (4) V-förmig angeordnet sind und daß die Stege (7) der zweiten Zellen (5) zweier gegenüberliegender Seiten V-förmig ausgebildet sind und daß die beiden anderen gegenüberliegenden Stege (8) bogenförmig aneinander angelegt sind.

2. Stent nach Anspruch 1, **dadurch gekennzeichnet, daß** die gefalteten Stege (6) der ersten Zellen (4) einen sich in Umfangsrichtung erstreckenden bandartigen Bereich bilden.

3. Stent nach Anspruch 1, **dadurch gekennzeichnet, daß** der bogenförmige Bereich im wesentlichen s-förmig ausgebildet ist.

4. Stent nach einem der Ansprüche 1 bis 3, **dadurch gekennzeichnet, daß** die ersten Zellen (4) auch nicht-gefaltete Stege (9) aufweisen die bogenförmig ausgestaltet sind.

5. Stent nach Anspruch 4, **dadurch gekennzeichnet, daß** die nicht-gefalteten Stege (9) der ersten Zellen jeweils zueinander gleich und parallel ausgebildet sind.

## Claims

1. A coronary stent (1) with a tubular, flexible body (2), the wall of which comprises a web structure, wherein the web structure comprises several mutually adjacent cells (4, 5) delimited by webs, which in each case are connected to adjacent cells via common webs, wherein first (4) and second (5) cells differing from one another are alternately disposed in the axial direction of the stent (1), wherein identical cells are provided in each case adjacent in the circumferential direction, wherein in the non-expanded state of the stent (1) the first cells (4) comprise folded webs (6) only on two opposite sides and wherein in the non-expanded state the second cells (5) are provided on all sides with web regions (7, 8) which are alternately folded in different manners and/or placed side by side,
**characterised in that** the folded webs (6) of the first cells (4) are disposed in a V-shape
and **in that** the webs (7) of the second cells (5) of two opposite sides have a V-shaped construction
and **in that** the two other opposite webs (8) are placed side by side in a curved shape.

2. A stent according to Claim 1,
**characterised in that** the folded webs (6) of the first cells (4) form a strip-shaped region extending in the circumferential direction.

3. A stent according to Claim 1,
**characterised in that** the curved region has a substantially S-shaped construction.

4. A stent according to one of Claims 1 to 3,
**characterised in that** the first cells (4) also comprise non-folded webs (9), which have a curved configuration.

5. A stent according to Claim 4,
**characterised in that** the non-folded webs (9) of the first cells are in each case constructed identically and parallel to one another.

## Revendications

1. Extenseur coronaire (1) avec un corps (2) flexible, tubulaire dont la paroi présente une structure de traverses, où la structure de traverses présente plusieurs cellules (4, 5) voisines, limitées par les traverses, lesquelles sont reliées aux cellules voisines, chaque fois par des traverses communes, dans lequel, dans la direction de l'axe de l'extenseur (1), sont disposées des premières (4) et deuxièmes (5) cellules alternées, différentes l'une de l'autre, dans lequel dans la direction du périmètre, des cellules voisines chaque fois similaires sont prévues, dans lequel les premières cellules (4) présentent à l'état non dilaté de l'extenseur (1), des traverses (6) plissées seulement sur deux côtés opposés, et dans lequel les deuxièmes cellules (5) sont munies à l'état non dilaté, sur tous les côtés, des domaines de traverse (7, 8) alternativement différents, plissés et/ou contigus, **caractérisé en ce que** les traverses plissées (6) des premières cellules (4) sont disposées en forme de V, et **en ce que** les traverses (7) des deuxièmes cellules (5) de deux côtés opposés ont la forme de V et que les deux autres traverses (8) opposées sont disposées l'un à côté de l'autre en forme d'arc.

2. Extenseur selon la revendication 1, **caractérisé en ce que** les traverses plissées (6) des premières cellules (4) forment un domaine de type ruban se prolongeant dans la direction du périmètre.

3. Extenseur selon la revendication 1, **caractérisé en ce que** le domaine en forme d'arc a essentiellement la forme de S.

4. Extenseur selon l'une des revendications 1 à 3, **caractérisé en ce que** les premières cellules (4) présentent également des traverses (9) non plissées, qui ont l'aspect d'arc.

5. Extenseur selon la revendication 4, **caractérisé en ce que** les traverses non plissées (9) des premières cellules sont formées de manière chaque fois similaire et parallèle l'une par rapport à l'autre.
